# EUROPÄISCHE PATENTSCHRIFT

(11) **EP 3 370 842 B1**
(45) Veröffentlichungstag und Bekanntmachung des Hinweises auf die Patenterteilung: **01.04.2020**
(21) Anmeldenummer: 16777593.1
(22) Anmeldetag: 23.09.2016
(51) Int. Cl.: B01D 3/14, C07C 263/20

(54) **DESTILLATIONSKOLONNE UND IHRE ANWENDUNG IN DER REINIGUNG VON ISOCYANATEN**
DISTILLING COLUMN, AND ITS USE IN THE PURIFICATION OF ISOCYANATES
COLONNE DE DISTILLATION ET SON UTILISATION DANS LE NETTOYAGE D'ISOCYANATES

(30) Priorität: 02.11.2015 EP 15192510
(43) Veröffentlichungstag der Anmeldung: 12.09.2018
(73) Patentinhaber: Covestro Deutschland AG, 51373 Leverkusen (DE)
(72) Erfinder: STEFFENS, Friedhelm, 51373 Leverkusen (DE); HEDDERICH, Wilfried, 40724 Hilden (DE); BAUSA, Jürgen, 51515 Kürten (DE); BEGGEL, Franz, 50935 Köln (DE); MICHELE, Volker, 51065 Köln (DE)
(74) Vertreter: Levpat
(86) Internationale Anmeldenummer: PCT/EP2016/072666
(87) Internationale Veröffentlichungsnummer: WO 2017/076551

(56) Entgegenhaltungen:
- EP-A1- 2 829 308
- EP-A2- 1 980 303
- EP-B1- 1 413 571
- US-A1- 2003 047 438
- US-A1- 2011 178 328

## Beschreibung

Die vorliegende Erfindung befasst sich mit einer Destillationskolonne zur Auftrennung eines Mehrstoffgemisches in mindestens 3 Fraktionen, wobei die Destillationskolonne über mindestens eine Trennwand verfügt und oberhalb der mindestens einen Trennwand mindestens ein Stoffaustauschelement (1) aufweist, wobei zwischen der mindestens einen Trennwand und dem mindestens einen Stoffaustauschelement (1) eine Vorrichtung (10) zur Vermischung der links und rechts der mindestens einen Trennwand nach oben strömenden Dampfströme angeordnet ist. Die vorliegende Erfindung befasst sich ebenfalls mit der Verwendung der erfindungsgemäßen Destillationskolonne in der Reinigung von Isocyanaten, insbesondere in der Reinigung von Toluylendiisocyanat (TDI).

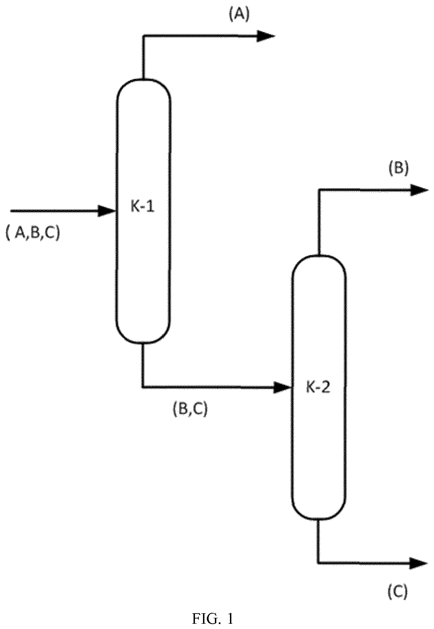

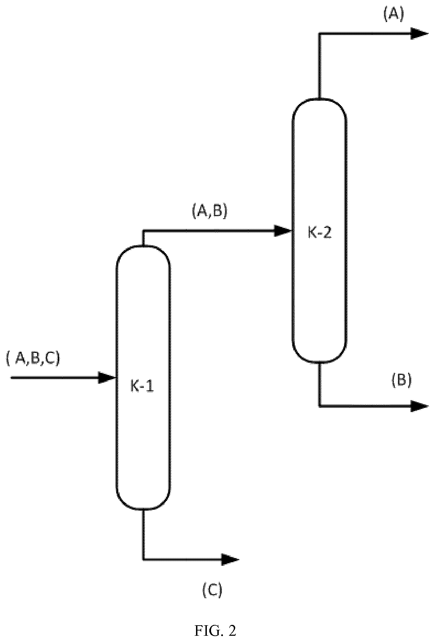

Soll ein Stoffgemisch bestehend aus mindestens einer leichtsiedenden Komponente (A), einer mittelsiedenden Komponente (B) und einer schwersiedenden Komponente (C) destillativ getrennt werden, sind bei Einsatz einfacher Destillationskolonnen ohne Trennwand in der Regel zwei Destillationskolonnen notwendig; vgl. Chemical Engineering and Processing, 49 (2010), 139 - 146. So kann in der ersten Destillationskolonne K1 (vgl. **FIG. 1**) der Leichtsieder (A) über Kopf abgetrennt werden. Im Sumpf wird ein von Leichtsieder freies Sumpfprodukt erzielt, welches dann in einer zweiten Kolonne K2 weiter aufgetrennt werden kann. Dabei werden die mittelsiedende Komponente (B) am Kopf und die schwersiedende Komponente (C) im Sumpf erhalten. In Abhängigkeit von der Zusammensetzung des zu trennenden Gemisches und der Trennbarkeit der einzelnen Komponenten (d. h. ihrer Siedepunktsunterschiede) kann auch zunächst der Schwersieder (C) im Sumpf der ersten Kolonne K1 abgetrennt werden (vgl. **FIG. 2**). Das am Kopf erhaltene Gemisch aus Leicht- und Mittelsieder (A, B) wird dann in der zweiten Kolonne K2 aufgetrennt. Die leichtsieden Komponente (A) wird dabei am Kopf und die mittelsiedende Komponente (B) im Sumpf erhalten.

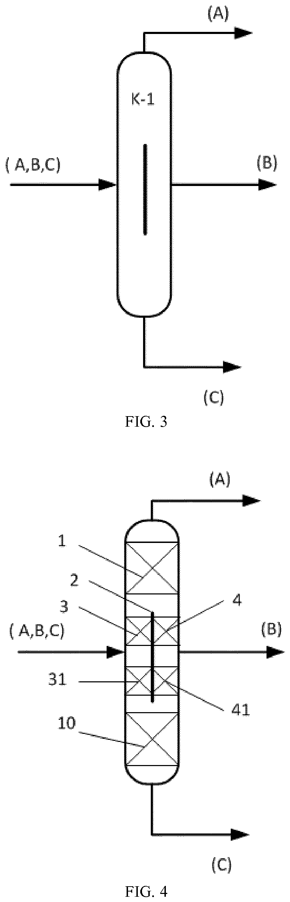

In vielen Fällen ist der Einsatz einer Destillationskolonne mit mindestens einer Trennwand (nachfolgend *Trennwandkolonne*) bezüglich der Investitions- und Energiekosten die günstigere Alternative zur klassischen Trennsequenz. Mit einer Trennwandkolonne können alle Komponenten in einem Schritt getrennt werden (vgl. **FIG. 3**). Ermöglicht wird dies durch eine im mittleren Teil der Kolonne angeordnete *Trennwand.* Das zu trennende Gemisch, auch Zulauf genannt, muss auf der entgegengesetzten Seite der Produktentnahme für die mittelsiedende Komponente zugegeben werden. **FIG. 4** zeigt eine typische Anordnung der Stoffaustausch-Elemente, wobei sehr häufig strukturierte Packungen zum Einsatz kommen. Oberhalb der Trennwand (2) ist ein gemeinsames Stoffaustauschelement (auch Packungs-Bett genannt) (1) angeordnet. Es dient zur Anreicherung der leichtsiedenden Komponente (A) durch Abtrennung der mittelsiedenden Komponente (B). Links und rechts der Trennwand sind die Stoffaustauschelemente (Packungs-Betten) 4, 41, 3 und 31 angeordnet. Bett 4 und 31 dienen zur Abtrennung der leichtsiedenden Komponente (A), sodass diese Komponente weder die Seitenstrom-Entnahme für die mittelsiedende Komponente (B) noch den Bereich unterhalb der Trennwand erreichen kann. Bett 3 und 41 dienen zur Abtrennung der schwersiedenden Komponente (C), mit dem Ziel, im Bereich oberhalb der Trennwand und an der Seitenstrom-Entnahme für die Komponente (B) möglichst frei von Komponente (C) zu sein. Das gemeinsam genutzte Bett 10 dient zur Konzentrierung der schwersiedenden Komponente (C).

Wie in Chemical Engineering, August 2014, 40 - 48 dargestellt, nimmt die Bedeutung der Trennwandkolonnen seit ca. 1985 stetig zu und umfasst heute neben unsymmetrisch angeordneten Trennwänden auch Anwendungen mit mehr als einer Trennwand. Zur weiteren Energieintegration sind Zwischen-Verdampfer und/oder Kondensatoren möglich, wie in WO 2010/039972 A2 beschrieben. Diese Anmeldung beschreibt ferner - vgl. FIG. 1 - die Anordnung von zwei Einbauten (124, 122 - *"trays"*) im Bereich oberhalb der Trennwand. Zwischen diesen beiden Einbauten befindet sich ein Produktauslass (128). Beide Einbauten befinden sich in einem Bereich der Trennwandkolonne, in welchem sich nach oben strömender Dampf und herunterrieselnde Flüssigkeit im Gegenstrom bewegen; es handelt sich also um *Stoffaustauschelemente* und nicht um Vorrichtungen zur Homogenisierung reiner Dampfströme.

EP 1 980 303 A2 befasst sich mit einem speziellen Rückflussteiler (2) für Trennwandkolonnen (1). Der Rückflussteiler (2) ist außerhalb der Kolonne (1) angeordnet (vgl. FIG. 1). Die Schrift offenbart zwei Ausführungsformen des Rückflussteilers (2); vgl. FIG. 2a und FIG. 2b. In beiden Varianten weist die Kolonne (1) oberhalb der Trennwand einen Flüssigkeitssammler (13) auf, welcher einen Boden (15) und Kamine (16) umfasst. Die Schrift offenbart nicht, dass im Bereich zwischen dem Ende der Trennwand und der Packung (12) eine besondere Homogenisierung des nach oben strömenden Dampfes stattfindet. Nach oben strömender Dampf kann in Kaminen nicht durchmischt werden, da diese die Aufgabe haben, durch Bereitstellung eines hohen freien Querschnitts den Druckverlust zu minimieren.

US 2003/0047438 A1 befasst sich mit speziellen Trennwandkolonnen. Die Abbildungen 1 und 21 zeigen mit Vorrichtung (54) und Lamellen (71) einen Flüssigkeitssammler. In Absatz [0052] wird die Wirkungsweise dieses Flüssigkeitssammlers beschrieben. Dort heißt es, dass die nach oben strömenden Dämpfe vom Kolonnenzentrum weggelenkt werden und trotzdem ein ausreichender Kontakt von Flüssigkeit und Dampf in den angrenzenden Stoffaustausch-Elementen gegeben ist. Eine Vorrichtung zur Homogenisierung nach oben strömender Dampfströme zwischen der Oberkante der Trennwand und einem Stoffaustauschelement wird in dieser Schrift nicht offenbart. Lamellenflüssigkeitssammler wie in dieser Schrift beschrieben (vgl. 54 und 71 in FIG. 3) eignen sich nicht zur Homogenisierung von Dampfströmen in Kolonnen.

EP 2 829 308 A1 befasst sich mit einem Flüssigkeitsverteiler (100) für Trennwandkolonnen. Abbildung 1 zeigt die Einrichtungen in einem solchen Flüssigkeitsverteiler innerhalb einer Trennwandkolonne direkt oberhalb der Trennwand (190). Als Einrichtungen des Flüssigkeitsverteilers sind u. a. ein Flüssigkeitssammler (130), der als Kaminboden (134, 136) ausgeführt ist, dargestellt. Durch die Vielzahl der Kamine ist der beschriebene Flüssigkeitssammler für eine Durchmischung von nach oben strömenden Dampfströmen ungeeignet.

Die vielfaltigen Möglichkeiten machen die Trennwandkolonnen-Technologie zu einer wichtigen Komponente, um den Energieverbrauch und die Herstellkosten in der Produktion zu reduzieren.

Eine typische Anwendung der Trennwandkolonnen-Technologie ist die Reinigung von Isocyanaten, insbesondere Toluylendiisocyanat (fortan TDI). Hier wird bei der chemischen Umsetzung des entsprechenden Amins (insbesondere Toluylendiamin, fortan TDA) mit Phosgen unter Verwendung eines inerten Lösungsmittels entweder als Verdünnungsmittel während der Reaktion und/oder als Quenchmedium zum raschen Reaktionsabbruch ein Rohprodukt erhalten, welches dann durch Destillation aufgearbeitet werden muss. Dabei wird im Fall von TDI in der Regel (vgl. EP 1 371 635 B1 und EP 1 413 571 B1) die Trennwandkolonne dazu genutzt, das TDI von leichtsiedenden Komponenten, vom Lösungsmittel und schwersiedenden Komponenten zu trennen, sodass es als verkaufsfähiges Produkt erhalten wird. So beschreibt EP 1 371 635 B1 eine Trennwandkolonne mit weniger als 2 % der leichtsiedenden Komponente Phosgen im Zulauf. Der Produktstrom wird im Bereich der Trennwand, auf der dem Zulauf abgewandten Seite, mit einer Reinheit von mindestens 99,5 Gew.-% EP 1 371 635 B1 entnommen. Er enthält darüber hinaus weniger als 200 Gew.-ppm Lösungsmittel und/oder chlorierte aromatische Kohlenwasserstoffe, weniger als 100 Gew.-ppm hydrolysierbares Chlor (HC) und weniger als 40 Gew.-ppm saure Anteile. Im Sumpf fällt ein mit schwersiedenden Komponenten angereicherter Stoffstrom an. In EP 1 413 571 B1 wird ein Verfahren zur Reinigung von TDI beschrieben bei dem das Zulaufgemisch zur Trennwandkolonne weniger als 20 Gew.-% Lösungsmittel enthält. Dieses Lösungsmittel wird am Kopf der Kolonne mit einer Reinheit von 20 bis 99 Gew.-% entnommen und enthält alle leichtsiedenden Komponenten. Die Anforderungen an die Reinheit des Produktstromes TDI ist analog zu der in EP 1 371 635 B1 beschriebenen. In EP 1 575 907 B1 wird ein Verfahren zur Reinigung von TDI in Kombination mit einer weiteren Konzentrierung der schwerer siedenden Komponenten beschrieben. Die bei der Konzentrierung der schwerer siedenden Komponenten anfallende TDI-Fraktion wird zwecks Rückgewinnung wieder der Trennwandkolonne zugeführt. Als Folge dessen besitzt diese Trennwandkolonne 2, gegebenenfalls 3 Zuläufe und je nach Anordnung eine bis in den Sumpf führende Trennwand.

Da das erhaltene TDI in der Regel dem Verkaufsprodukt entspricht, unterliegt es hohen Anforderungen an die Einhaltung der Qualität. Jede Fehlfunktion der Trennwandkolonne führt zu nicht spezifikationsgerechtem Produkt. Auch die anderen erhaltenen Ströme müssen die geforderten Spezifikationen hinsichtlich ihrer Zusammensetzung erreichen, da sie zwecks Wiederverwendung häufig in den Produktionsprozess zurückgeführt werden. Werden diese Spezifikationen nicht erreicht, kann dies zu ineffizient hohen Kreislaufströmen oder zu Ausbeuteverlusten führen, insbesondere dann, wenn zum Beispiel im Falle der Herstellung von Isocyanaten und insbesondere TDI das wiedergewonnene Lösungsmittel nicht der geforderten Reinheit für einen Einsatz in der Reaktionsstufe erreicht. Dies stellt hohe Anforderungen an die Dimensionierung der Kolonne sowie an ihre Fertigung.

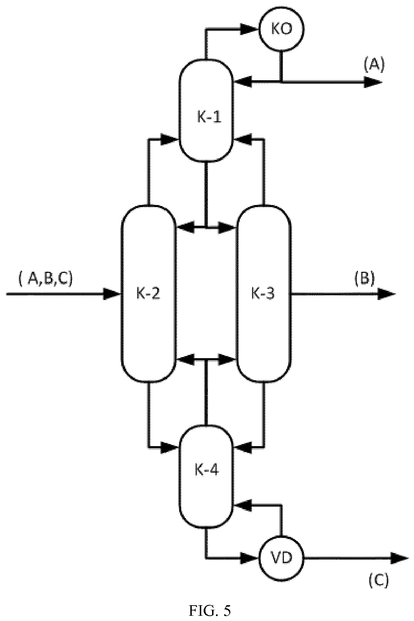

Die Dimensionierung von Trennwandkolonnen erfolgt in der Regel durch stationäre Simulatoren wie sie zum Beispiel von der Firma AspenTech kommerziell angeboten werden. Im Allgemeinen kann die Simulation durch die Verschaltung von herkömmlichen Kolonnen erfolgen (vgl. Chr. Hiller "Auslegung von Trennwandkolonnen: Modellierungsansätze und experimentelle Validierung", Kapitel 2 des Vortragsskripts des Trennwandkolonnen-Symposiums vom 13. Oktober 2011 am Institut für Prozess- und Anlagentechnik der Technischen Universität Hamburg-Harburg). **FIG. 5** zeigt eine solche Anordnung. Kolonne 1 (K-1) stellt dabei den Teil oberhalb Trennwand dar. Den Bereich der Trennwand wird durch zwei weitere, separate Kolonnen (K-2, K-3) abgebildet. K-4 spiegelt den Bereich unterhalb der Trennwand wieder. Die zu der Kolonne gehörenden Apparate wie Kondensator und Verdampfer werden vorteilhaft als separate Modelle beschrieben, können aber auch, je nach verwendetem Simulationsprogramm, in die Kolonnenmodelle K-1 und K-4 integriert werden. Durch verbindende Stoffströme werden die einzelnen Kolonnen K-1 bis K-4, der Kondensator und der Verdampfer zu einer Trennwandkolonne. Als Kolonnenmodelle können Gleichgewichts-Stufenmodelle oder aber Nichtgleichgewichts-Stufenmodelle eingesetzt werden. Hierbei sind die Gleichgewichts-Stufenmodelle der einfachere und flexiblere Ansatz, da sie weniger Stoffdaten benötigen. Diese Modelle stoßen aber bei stark nicht-idealen Stoffsystemen an ihre Grenzen.

Wesentlich für die Funktionstüchtigkeit einer Trennwandkolonne ist die Aufteilung der Brüden aus dem unteren gemeinsamen Teil K-4 auf die beiden Seiten der Trennwand K-2 und K-3. Diese Aufteilung ist nur abhängig vom Verhältnis der Druckverluste links und rechts der Trennwand. Bei realen Kolonnen ist der Druckverlust abhängig von den gewählten Kolonneneinbauten sowie der Gas- und Flüssigkeitsbelastung. Für die Simulation als Basis für eine Dimensionierung müssen also Prozessmodelle Verwendung finden, die den Druckverlust als Funktion der Hydrodynamik der später Verwendung findenden Kolonneneinbauten berechnen können. Nur dann ist eine gesicherte Vorhersage der Trennleistung der Kolonne, speziell für Teillastverhalten und geänderte Betriebsparameter, wie die Veränderung der Zusammensetzung des Zulaufes, möglich.

Die Aussagekraft der Simulation sollte durch experimentelle Daten nachgewiesen werden, besonders dann wenn die Beschreibung der Stoffdaten nicht mit der nötigen Genauigkeit möglich ist oder aber Produkteigenschaften erreicht werden müssen, die sich einer Simulation entziehen (z. B. Farbe). Untersuchungen (vgl. G. Niggemann et al., Ind. Eng. Res. 2010, 49, 6566 - 6577) ergaben eine gute Übereinstimmung von Labor- und Simulationsergebnissen, insbesondere hinsichtlich der Gasverteilung unterhalb der Trennwand. Die verwendete Testeinrichtung bestand aus einer Destillationskolonne mit einem Innendurchmesser von 68 mm und beinhaltete mehrere Stoffaustauschelemente vom Typ Montz B1-500. Neben der guten Übereinstimmung von experimentellen und rechnerischen Ergebnissen berichtet diese Veröffentlichung über den Einfluss des Wärmetransportes auf das Trennverhalten der Kolonne. Dieser Einfluss wird jedoch auf Laborkolonnen beschränkt, da hier die Oberfläche der Trennwand relativ zum Durchsatz viel größer ist als bei technischen Kolonnen. Auch eine andere Arbeit (Chr. Hiller et al., Heat Mass Transfer (2010) 46, 1209 - 1220) bestätigt unter Verwendung von Nicht-Gleichgewichts Modellen eine gute Übereinstimmung von experimentellen Daten mit den Simulationsergebnissen.

Für die technische Ausführung von Trennwandkolonnen bieten verschiedene Hersteller (z. B. Sulzer Chemtech, Montz) die entsprechenden Kolonneneinbauten wie Flüssigkeitssammler und Wiederverteiler an. Die Trennwand kann entweder mit dem äußeren Kolonnenmantel verschweißt oder aber lose gesteckt werden, wie in EP 1 008 577 A1 beschrieben.

Zusammenfassend bleibt festzuhalten, dass die Auslegung von Trennwandkolonnen für großtechnische Einsatzzwecke im Stand der Technik üblicherweise auf Simulationen beruht, deren Übereinstimmung mit experimentellen Daten im Allgemeinen als ausreichend gut angesehen wird. In der betrieblichen Praxis hat sich jedoch überraschenderweise gezeigt, dass dies nicht immer der Fall ist. So wurde beobachtet, dass etwa die Reinheit von als Seitenstrom im Bereich der Trennwand entnommenem TDI in der Realität von der durch Simulationsergebnisse zu erwartenden Reinheit zeitweise signifikant abweichen kann. Gleiches gilt für die Reinheit des als leichtsiedende Komponente am Kopf der Trennwandkolonne entnommenen Lösungsmittels. Es bestand daher ein Bedarf an weiteren Verbesserungen existierender Trennwandkolonnen.

Diesem Bedarf Rechnung tragend ist ein Gegenstand der vorliegenden Erfindung eine Destillationskolonne zur Auftrennung eines Mehrstoffgemisches in mindestens 3 Fraktionen, wobei die Destillationskolonne über mindestens eine Trennwand (2) verfügt und oberhalb der mindestens einen Trennwand mindestens ein Stoffaustauschelement (1), in dem ein Stoffaustausch zwischen nach oben strömendem Dampf und nach unten strömender Flüssigkeit stattfindet, aufweist, wobei zwischen der mindestens einen Trennwand (2) und dem mindestens einen Stoffaustauschelement (1), im Falle des Vorhandenseins mehrerer Stoffaustauschelemente (1) zwischen der mindestens einen Trennwand (2) und dem untersten Stoffaustauschelement (1), eine Vorrichtung (10) zur Vermischung der links und rechts der mindestens einen Trennwand (2) nach oben strömenden Dampfströme angeordnet ist, wobei sich die Vorrichtung (10) in einem Bereich der Destillationskolonne, in dem die Dampfströme aus dem Bereich der mindestens einen Trennwand (2) nach oben strömen, ohne dabei auf herabströmende Flüssigkeit zu treffen, befindet, was dadurch realisiert wird, dass unterhalb des mindestens einen Stoffaustauschelements (1), im Falle des Vorhandenseins mehrerer Stoffaustauschelemente (1) unterhalb des untersten Stoffaustauschelements (1), ein Flüssigkeits-Sammler (7) zum Auffangen der aus dem mindestens einen Stoffaustauschelement (1) ablaufenden Flüssigkeit angeordnet ist, wobei der Flüssigkeits-Sammler (7) so ausgestaltet ist, dass die in diesem Flüssigkeits-Sammler (7) aufgefangene Flüssigkeit in einen Ringkanal (8) weitergeleitet und von dort
über einen Austrittsstutzen aus dem Inneren der Destillationskolonne heraus über außerhalb des Kolonnenkörpers befindliche Leitungen (11)
oder
über innerhalb des Kolonnenkörpers befindliche Leitungen
auf im Bereich der mindestens einen Trennwand (2) angeordnete Flüssigkeitsverteiler (5) und (6) gegeben wird.

Ein weiterer Gegenstand der vorliegenden Erfindung ist die Verwendung der erfindungsgemäßen Destillationskolonne zur Reinigung von Isocyanaten, insbesondere zur Reinigung von Toluylendiisocyanat (TDI). Anders ausgedrückt ist ein weiterer Gegenstand der vorliegenden Erfindung ein Verfahren zur Reinigung von Isocyanaten, insbesondere zur Reinigung von Toluylendiisocyanat (TDI), bei dem eine erfindungsgemäße Destillationskolonne eingesetzt wird.

Erfindungsgemäß weist die Destillationskolonne mindestens eine *Trennwand (2)* auf, es handelt sich also um eine *Trennwandkolonne.* Die mindestens eine Trennwand ist vertikal im Innenraum der Destillationskolonne angeordnet. Bevorzugt weist die erfindungsgemäße Destillationskolonne maximal 2 Trennwände auf, ganz besonders bevorzugt 1 (d. h. genau eine) Trennwand. Im Fall von mehr als einer Trennwand sind die Trennwände in Bezug auf die Längsachse des Kolonnenkörpers nebeneinander angeordnet und können unterschiedliche Höhen haben. Bevorzugt, jedoch nicht zwingend, ist die mindestens eine Trennwand symmetrisch angeordnet, d. h. im Fall einer einzigen Trennwand befindet sich diese in der Mitte des Querschnitts des Kolonnenkörpers. Im Fall mehrerer Trennwände bezieht sich der Ausdruck *"links und rechts der mindestens einen Trennwand nach oben strömenden Dampfströme"* auf die entsprechenden Dampfströme aller vorhandenen Trennwände.

*Oberhalb der mindestens einen Trennwand (2)* befindet sich mindestens ein *Stoffaustauschelement* (1). Sind mehrere Trennwände unterschiedlicher Höhe vorhanden, so befindet sich das mindestens eine Stoffaustauschelement (1) oberhalb der höchsten Trennwand. Bevorzugt weist die erfindungsgemäße Destillationskolonne maximal 2 Stoffaustauschelemente oberhalb der mindestens einen Trennwand auf, ganz besonders bevorzugt 1 (d. h. genau ein) Stoffaustauschelement (1) oberhalb der mindestens einen Trennwand. Sind mehrere Stoffaustauschelemente (1) vorhanden, so sind diese übereinander angeordnet. Bevorzugt reicht das mindestens eine Stoffaustauschelement (1), im Falle des Vorhandenseins mehrerer Stoffaustauschelemente (1) mindestens das unterste Stoffaustauschelement (1), über den gesamten Querschnitt des Kolonnenkörpers. In dem mindestens einen Stoffaustauschelement (1) oberhalb des Trennwandbereichs findet ein Stoffaustausch zwischen nach oben strömendem Dampf und nach unten strömender Flüssigkeit statt. Das mindestens eine Stoffaustauschelement (1) befindet sich also in einem Bereich der Destillationskolonne, in dem Dampf- und Flüssigphasen im Gegenstrom aufeinander treffen (im Gegensatz zur Mischvorrichtung (10), wie weiter unten näher ausgeführt wird).

Erfindungsgemäß befindet sich zwischen der mindestens einen Trennwand und dem mindestens einen Stoffaustauschelement (1) *eine Vorrichtung (10) zur Vermischung der links und rechts der mindestens einen Trennwand nach oben strömenden Dampfströme.* Dabei kann diese Vorrichtung (10) im Sinne der Erfindung statische und/oder bewegliche Mischelemente umfassen, wie weiter unten noch näher ausgeführt wird. Diese Mischvorrichtung (10) befindet sich in einem Bereich der Destillationskolonne, in dem die Dampfströme aus dem Bereich der mindestens einen Trennwand (2) nach oben strömen, ohne dabei auf herabströmende Flüssigkeit zu treffen. Daher bewirkt die Mischvorrichtung (10) eine Homogenisierung der Dampfströme ohne Stoffaustausch.

Überraschend wurde gefunden, dass die oben geschilderten Probleme im Zusammenhang mit der Reinheit der Produktströme durch den Einbau einer solchen Vorrichtung gelöst oder zumindest verringert werden können. Ohne auf eine Theorie festgelegt sein zu wollen wird angenommen, dass ohne eine solche Vorrichtung in Trennwandkolonnen mit mindestens einem Stoffaustauschelement oberhalb der Trennwand (dem Stoffaustauschelement (1)) die Durchmischung der Dampfströme, welche links und rechts aus dem Trennwandbereich aufsteigen, *nicht ideal* ist. Diese nicht ideale Durchmischung führt zu einer bezüglich der Dampfgeschwindigkeit und Dampfzusammensetzung ungleichmäßigen Anströmung des Stoffaustauschelementes oberhalb der Trennwand und somit zu einem Verlust an Trennleistung. Dabei wird die Durchmischung der Dampfströme umso schlechter, je größer der Kolonnendurchmesser ist. Dies liegt an der zur Verfügung stehenden Höhe zwischen der Oberkante der Trennwand und dem darüber befindlichen Stoffaustauschelement, welche bei steigendem Kolonnendurchmesser relativ gesehen immer kleiner wird. Die vorliegende Erfindung ermöglicht daher eine Homogenisierung der Dampfströme (auch Brüdenströme oder kurz Brüden genannt) nach Austritt aus dem Trennwandbereich vor Eintritt in das erste Stoffaustauschelement oberhalb des Trennwandbereichs (d. i. das in der Terminologie dieser Erfindung als Stoffaustauschelement (1) bezeichnete). Durch diese Homogenisierung wird die Trennleistung der Kolonne erhöht, sodass die der Dimensionierung der Kolonne zugrunde liegenden Produktspezifikationen wieder eingehalten werden können.

Die Erfindung im Allgemeinen und verschiedene Ausführungsformen der Erfindung im Besonderen werden nachfolgend anhand der Abbildungen näher beschrieben. Verschiedene Ausführungsformen sind dabei beliebig miteinander kombinierbar, sofern sich für den Fachmann aus dem technischen Gesamtzusammenhang nicht das Gegenteil ergibt.

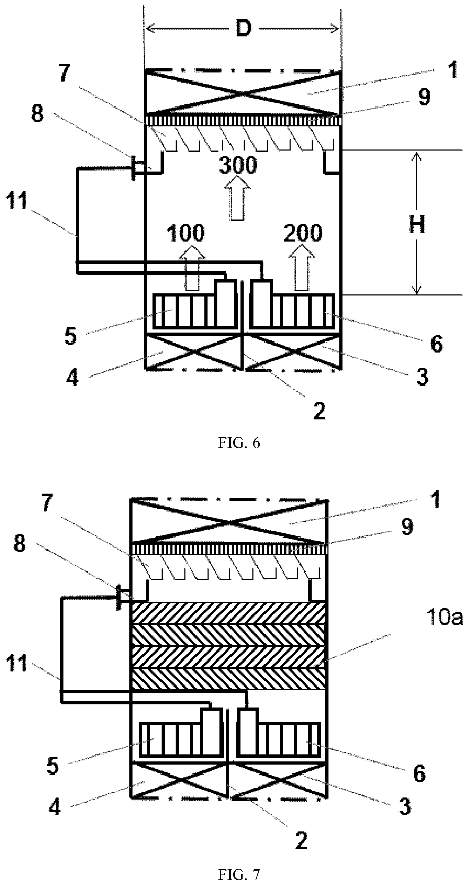

**FIG. 6** zeigt den Bereich zwischen oberem Ende der Trennwand (2) und dem nächst höherem Stoffaustauschelement (1) in einer herkömmlichen Destillationskolonne ohne die erfindungsgemäße Vorrichtung zur Homogenisierung der Brüdenströme (10). Am oberen Ende der Trennwand (2) befinden sich die Stoffaustauschelemente (3) und (4) sowie die dazu gehörigen Flüssigkeitsverteiler (5) und (6) jeweils links und rechts der Trennwand. Das Stoffaustauschelement (1) liegt auf einem Tragrost (9) auf. Die aus dem Stoffaustauschelement (1) ablaufende Flüssigkeit wird zur Wiederverteilung von einem Flüssigkeits-Sammler (7) aufgefangen und in den Ringkanal (8) mit Austrittsstutzen weitergeleitet und von dort über außerhalb des Kolonnenkörpers befindliche Leitungen (11) auf die Flüssigkeitsverteiler (5) und (6) gegeben. Aus dem Trennwandbereich treten links und rechts die Brüdenströme (Dampfströme) m_{L} (100) und m_{R} (200) mit den Geschwindigkeiten w_{L} und w_{R} sowie den Konzentrationen x_{L} und x_{R} aus. Um die maximale Trennleistung im Stoffaustauschelement (1) zu erreichen, ist eine Homogenisierung dieser Brüdenströme nötig, mit dem Ziel, einen vermischten Gesamtstrom m_{G} (300) mit einer Mischkonzentration xₘ und einer gleichmäßige Brüdengeschwindigkeit wₘ über den gesamten Querschnitt der Kolonne vor Eintritt in das Stoffaustauschelement (1) zu erreichen. Hierzu steht die Höhe H zur Verfügung.

Erfindungsgemäß wird zur Erreichung dieses Ziels die Vorrichtung (10) zur Vermischung der links und rechts der mindestens einen Trennwand nach oben strömenden Dampfströme verwendet. Aufgabe dieser Mischvorrichtung (10) ist eine Homogenisierung der nach oben strömenden Dampfströme, bevor diese im Bereich des mindestens einen Stoffaustauschelements (1) mit herabströmender Flüssigkeit in Kontakt treten. Die Mischvorrichtung (10) befindet sich daher in einem Bereich der Destillationskolonne, in dem keine herabströmende Flüssigkeit aus dem oberen Bereich der Destillationskolonne auftritt. Ein solcher Bereich wird in vorrichtungstechnischer Hinsicht dadurch realisiert, dass in der erfindungsgemäßen Destillationskolonne unterhalb des mindestens einen Stoffaustauschelements (1), im Falle des Vorhandenseins mehrerer Stoffaustauschelemente (1) unterhalb des untersten Stoffaustauschelements (1), ein Flüssigkeits-Sammler (7) zum Auffangen der aus dem mindestens einen Stoffaustauschelement (1) ablaufenden Flüssigkeit angeordnet wird. Dieser Flüssigkeits-Sammler (7) ist so ausgestaltet, dass die in diesem aufgefangene Flüssigkeit so in den Bereich der mindestens einen Trennwand (2) geleitet, d. h. auf im Bereich der mindestens einen Trennwand (2) angeordnete Flüssigkeitsverteiler (5) und (6) gegeben wird, dass im Bereich zwischen der mindestens einen Trennwand (2) und dem mindestens einen Stoffaustauschelement (1), im Falle des Vorhandenseins mehrerer Stoffaustauschelemente (1) zwischen der mindestens einen Trennwand (2) und dem untersten Stoffaustauschelement (1), keine herabströmende Flüssigkeit aus dem oberen Bereich der Destillationskolonne auftritt. Zu diesem Zweck wird die in dem Flüssigkeits-Sammler (7) aufgefangene Flüssigkeit in einen unterhalb des Flüssigkeits-Sammlers (7) angeordneten Ringkanal weitergeleitet.

In der in FIG. 6 gezeigten Destillationskolonne befindet sich ein solcher Bereich ohne herabströmende Flüssigkeit oberhalb der Trennwand (2) und unterhalb des Ringkanals (8). In diesen Bereich wird die erfindungsgemäße Mischvorrichtung (10) bevorzugt eingebaut, wie in **FIG. 7** am Beispiel einer Mischerpackung (10a) gezeigt. Dass in diesem Bereich keine herabströmende Flüssigkeit mit dem nach oben strömenden Dampf in Kontakt tritt wird in der in FIG. 7 gezeigten Ausführungsform erreicht durch eine Ausgestaltung der erfindungsgemäßen Destillationskolonne derart, dass unterhalb des Stoffaustauschelements (1) ein Flüssigkeits-Sammler (7) zum Auffangen der aus dem mindestens einen Stoffaustauschelement (1) ablaufenden Flüssigkeit angeordnet ist, wobei der Flüssigkeits-Sammler (7) wiederum so ausgestaltet ist, dass die in diesem aufgefangene Flüssigkeit in einen unterhalb des Flüssigkeits-Sammlers (7) angeordneten Ringkanal (8) weitergeleitet und von dort über einen Austrittsstutzen aus dem Inneren der Destillationskolonne heraus über außerhalb des Kolonnenkörpers befindliche Leitungen (11) auf im Bereich der mindestens einen Trennwand (2) angeordnete Flüssigkeitsverteiler (5) und (6) gegeben wird. Diese Ausgestaltung der Destillationskolonne ist natürlich nicht auf Mischerpackungen (10a) beschränkt, sondern auch auf andere Mischvorrichtungen (10), wie sie im Folgenden näher erläutert werden, anwendbar. Sie ist ebenfalls anwendbar auf Destillationskolonnen mit mehreren Stoffaustauschelementen (1) oberhalb der mindestens einen Trennwand (2). Ferner ist es auch möglich, die Leitungen zur Abfuhr der Flüssigkeit aus dem Ringkanal (8) im Inneren der Destillationskolonne zu verlegen. Wenn in dieser Ausführungsform das mindestens eine Stoffaustauschelement (1) über den ganzen Querschnitt der Destillationskolonne reichen soll, was wie bereits erwähnt grundsätzlich bevorzugt ist, so ist das mindestens eine Stoffaustauschelement (1) mit Aussparungen für die Leitungen zur Abfuhr der Flüssigkeit aus dem Ringkanal (8) versehen. Die in FIG. 7 gezeigte Anordnung mit außen liegenden Leitungen (11) ist jedoch bevorzugt, weil hierdurch der Einbau der Mischvorrichtung (10) nicht behindert wird. Außerdem ist bei dieser Anordnung mit außen liegenden Leitungen (11) die gezielte Verteilung der Flüssigkeit auf die Flüssigkeitsverteiler (5) und (6) einfacher zu realisieren.

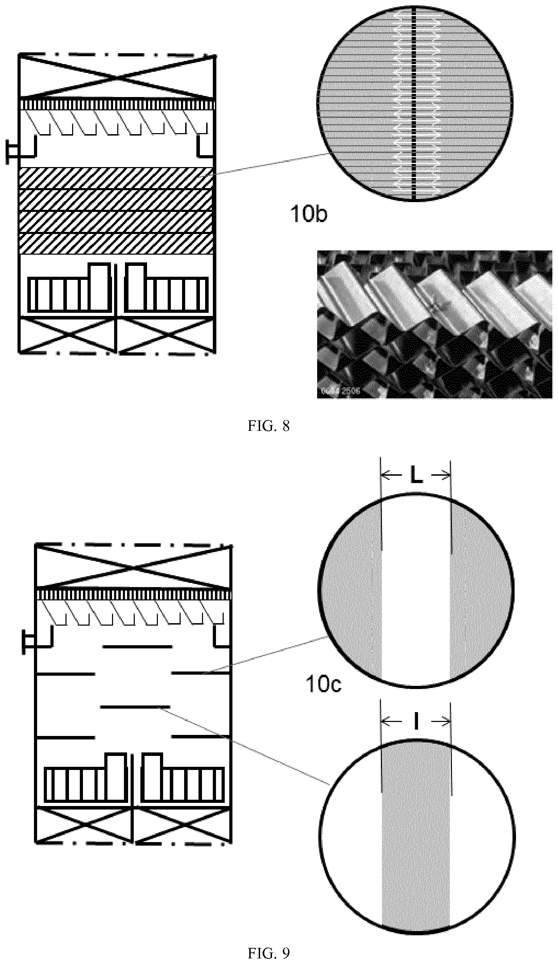

Im Sinne der Erfindung sind als Mischvorrichtung (10) zunächst alle kommerziell erhältlichen Gasmischelemente geeignet, zum Beispiel statische Mischer wie sie von verschiedenen Firmen angeboten werden und beispielsweise in Chemineer TECH NEWS, Kenics Statik-Mischer, Chemineer Ltd., 1994 und SULZER CHEMTECH, Misch- und Reaktionstechnik*,* beschrieben sind. Die bereits erwähnten Mischerpackungen (10a) sind beispielsweise in SULZER CHEMTECH, Misch- und Reaktionstechnik beschrieben (etwa die Sulzer-Mischerpackung "SMV/SMVP"; FIG. 7). Die Mischerpackung (10a) kann dabei wie das Stoffaustausch-Element (1) auf einem Tragrost gelagert werden. **FIG. 8** zeigt ein Mischelement (10b), das aus gewellten Blechlagen - in der gezeigten Ausführungsform ohne Löcher, was bevorzugt ist - besteht, wobei im Gegensatz zu Stoffaustausch-Elementen die einzelnen Lagen nicht verdreht werden. Die dadurch entstehenden Kanäle führen die Brüden abwechselnd von links nach rechts bzw. von rechts nach links.

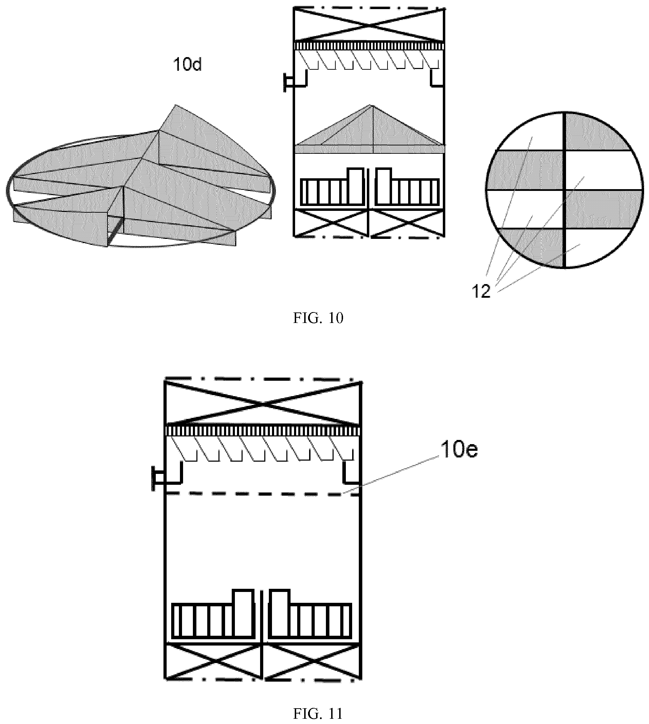

Erfindungsgemäß kann das Misch-Element (10) auch aus Umlenkblechen (10c) wie in **FIG. 9** dargestellt realisiert werden. Dabei kann für die Maße "L" und "1" L = 1 gelten, aber auch L kleiner oder größer 1 ist möglich. Je nach Mischaufgabe können die Bleche auch ganz oder teilweise gelocht sein. Wie in FIG. 9 dargestellt, sind bei dieser Anordnung die Umlenkbleche horizontal ausgerichtet. Im Gegensatz dazu sind auch Umlenkbleche mit einer Steigung zu Mitte der Kolonne hin möglich (10d). **FIG 10** zeigt diese Möglichkeit.

Um das obere Stoffaustauschelement (1) mit möglichst gleichmäßiger Brüdengeschwindigkeit anzuströmen, kann/können im Sinne der Erfindung auch ein Siebboden oder mehrere Siebböden (10e) gemäß **FIG. 11** eingesetzt werden. Alternativ sind Kaminböden (10f), Glockenböden (10g) und Ventilböden (10h) geeignete Einbauten zur Homogenisierung der Brüdengeschwindigkeit.

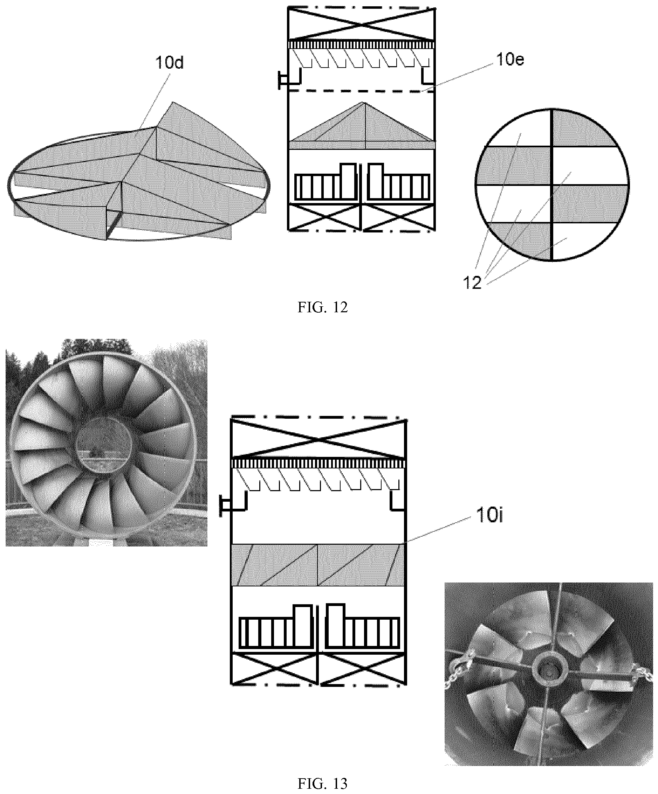

Alle beschriebenen Mischelemente (10) können auch mit einander kombiniert werden. **FIG. 12** zeigt die Kombination von schrägen Umlenkblechen (10d) zur Quervermischung der Brüden mit einem Siebboden (10e) zur Homogenisierung der Brüdengeschwindigkeit. Im rechten Teil der Abbildung ist ein Querschnitt der im linken Teil der Abbildung gezeigten schrägen Umlenkbleche 10d gezeigt. Die weißen Flächen (12) sind dabei offen.

Statoren (10i) sind ebenfalls geeignete Mischelemente (10) im Sinne der Erfindung und können feststehend oder drehend eingesetzt werden. **FIG. 13** stellt schematisch diese Lösung dar.

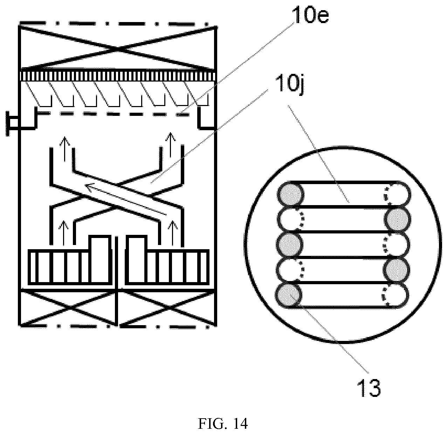

Im Sinne der Erfindung kann die Durchmischung der Brüdenströme auch durch Rohre (10j) erfolgen, die Brüdenanteile von links nach rechts bzw. in umgekehrter Richtung umlenken. In **FIG. 14** ist diese Mischeinrichtung (10j) in Kombination mit einem Siebboden (10e) dargestellt. Im rechten Teil der Abbildung ist eine Draufsicht von 10j mit angezeigtem oberem Brüdenaustritt (13) dargestellt. Anstelle von Rohren (10j) können auch Kanäle (10k) mit rechteckigem, quadratischem oder ovalem Querschnitt zum Einsatz kommen. Auch nach unten offene Kanäle mit beliebigem Querschnitt sind im Sinne der Erfindung für die Mischaufgabe geeignet. Ebenfalls geeignet sind Lochbleche (10l).

Geeignete Stoffaustauschelemente (1) sind dem Fachmann bekannt. Bevorzugt werden Füllkörperschüttungen, strukturierte Stoffaustausch-Packungen, Glockenböden, Ventilböden, Siebböden oder Kombinationen der vorgenannten Vorrichtungen eingesetzt. Im Gegensatz zur Mischvorrichtung (10) treffen im Bereich des Stoffaustauschelements (1) dampfförmige und flüssige Ströme im Gegenstrom aufeinander; es findet also tatsächlich ein *Stoffaustausch* zwischen Dampf- und Flüssigphase statt. Im Gegensatz dazu dient die Mischvorrichtung (10) nur der Homogenisierung der nach oben strömenden Dampfströme. Die Vorrichtungen (1) und (10) dienen also unterschiedlichen Zwecken, selbst wenn sie sich in apparativer Hinsicht gleichen sollten.

Grundsätzlich ist die erfindungsgemäße Destillationskolonne zur Auftrennung beliebiger Mehrstoffgemische (A, B, C) enthaltend mindestens 3 Stoffe mit unterschiedlichen Siedepunkten geeignet, wobei im Folgenden die Komponente mit dem niedrigsten Siedepunkt (sog. "Leichtsieder") mit (A), die mit dem höchsten Siedepunkt (sog. "Schwer- oder Hochsieder") mit (C) und die Komponente mit einem dazwischen liegenden Siedepunkt (sog. "Mittelsieder") mit (B) bezeichnet wird. Besonders bevorzugt ist die Verwendung der erfindungsgemäßen Destillationskolonne zur Reinigung von Isocyanaten. Diese Anwendung wird nachfolgend näher beschrieben. Dem Fachmann ist es ein leichtes, die dort gemachten Ausführungen auf die Anwendung in anderen technischen Gebieten zu übertragen.

Ein weiterer Gegenstand der vorliegenden Erfindung ist daher die Verwendung der erfindungsgemäßen Destillationskolonne zur Reinigung von Isocyanaten, insbesondere zur Reinigung von Toluylendiisocyanat (TDI). Weitere geeignete Isocyanate sind Methylendiphenyldiisocyanat (MDI), Hexamethylendiisocyanat (HDI), Pentamethylendiisocyanat (PDI), Isophorondiisocyanat (IPDI), m-Xylylendiisocyanat (XDI) und Dicyclohexylmethandiisocyanat ("H12-MDI"; auch bekannt unter dem Handelsnamen "Desmodur W" der Covestro Deutschland AG). Der zu reinigende Roh-Isocyanatstrom umfasst dabei "leichtsiedende" Anteile (A) wie insbesondere Lösungsmittel und ggf. Spuren an Phosgen und/oder Chlorwasserstoff und "schwersiedende" Anteile (C) wie polymerisierte Isocyanatfraktionen. Die Begriffe "leichtsiedend" und "schwersiedend" sind dabei in Relation zum Siedepunkt des angestrebten Wertprodukts, des Isocyanats, zu sehen. Dieses wird als sog. "Mittelsieder" (B) im Bereich der Trennwand als Seitenstrom entnommen. Die "Leichtsieder" (A) werden als Kopfstrom und die "Schwersieder" (C) als Sumpfstrom der Kolonne entnommen, wie in FIG. 4 schematisch für eine herkömmliche Trennwandkolone gezeigt (bezüglich der Entnahmestellen unterscheiden sich erfindungsgemäße Trennwandkolonnen nicht von herkömmlichen). Der Roh-Isocyanatstrom kann einem beliebigen Verfahren zur Herstellung von Isocyanaten aus dem Stand der Technik entstammen. Exemplarisch seien WO 2015/144658 (A1), EP 0792 263 (B1), EP 0 570 799 (B1) und EP 0 289 840 (B1) genannt.

Der in die erfindungsgemäße Destillationskolonne einzuspeisende Roh-Isocyanatstrom ist bevorzugt ein solcher, aus dem die Hauptmenge des Lösungsmittels, Phosgen und Chlorwasserstoff bereits in vorgelagerten Reinigungsstufen abgetrennt wurden. Solche vorgelagerten Reinigungsstufen sind aus dem Stand der Technik bekannt und bspw. beschrieben in EP 1 546 091 (B1), US 5,136,087, EP 1 854 783 (A2) und EP 2 210 873 (A1). Die erfindungsgemäße Destillationskolonne eignet sich in besonderer Weise zur Abtrennung polymerer Isocyanat-Fraktionen und Feinreinigung des angestrebten Isocyanats in einem Schritt (insbesondere im Fall von TDI). Sie ist jedoch auch zur Isomerentrennung geeignet (insbesondere im Fall der Auftrennung der einzelnen MDI-Isomeren).

Somit stellt Erfindung eine einfache Lösung für die eingangs geschilderten Probleme bereit. Die Erfindung ist auch auf bereits bestehende Trennwandkolonne anwendbar, weil sich die Vorrichtung (10) auch nachträglich einbauen lässt. Nachfolgende Beispiele illustrieren die Erfindung weiter.

### Beispiele

### Beispiel 1 (Vergleich - ohne Vorrichtung (10))

Ein Roh-TDI-Gemisch bestehend aus 15 Gew.-% leichtsiedenden Komponenten (überwiegend Lösungsmittel ortho-Dichlorbenzol, daneben Spuren an Phosgen und Chlorwasserstoff), 1 Gew.-% schwerer siedenden Komponenten (polymerisierte TDI-Fraktionen und Nebenkomponenten mit einem Siedepunkt oberhalb von TDI) und 84 Gew.-% mittelsiedenden Komponenten (das Zielprodukt TDI) wird in drei Ströme aufgetrennt. Die durch Simulationen und Laborergebnisse erwarteten Produktspezifikationen sind:
(A) Ein Kopfprodukt mit einem Gehalt von > 98 Gew.-% leichtsiedenden Komponenten.
(B) Ein Seitenstrom mit < 5 Gew.-ppm leichtsiedenden Komponenten.
(C) Ein Stoffstrom (Sumpfprodukt) mit < 15 Gew.-% schwerer siedende Komponenten.

In der betrieblichen Praxis erfolgt die Trennung in einer Trennwandkolonne mit einem Durchmesser von 3200 mm und bei einem Druck am Kopf der Kolonne von 70 mbar. Im Gegensatz zu den Simulations- und Laborergebnissen wird ein Kopfprodukt (A) erhalten, das 78 Gew.-% leichtsiedende Komponenten enthält. Der Seitenstrom (B) enthält zwischen 5 und 10 Gew.-ppm leichtsiedende Komponenten. Der Sumpfstrom (C) enthält zwischen 8 Gew.-% und 15 Gew.-% schwerer siedende Komponenten.

### Beispiel 2 (erfindungsgemäß - mit Vorrichtung (10))

Die in Beispiel 1 beobachtete Abweichung konnte durch spezielle Simulationsrechnungen auf eine unzureichende Homogenisierung der Dampfströme am Austritt der Trennwand zurückgeführt werden. Die Simulation aus Beispiel 1 wurde so geändert, dass die Vorrichtung (10) berücksichtigt wird. Nun werden bei einem Kopfdruck von 70 mbar spezifikationsgerechte Seitenströme erhalten. So wird ein Kopfprodukt (A) erhalten, das 98 Gew.-% leichtsiedende Komponenten enthält. Der Seitenstrom (B) enthält nur zwischen 1 und 5 Gew.-ppm leichtsiedende Komponenten. Der Sumpfstrom (C) enthält bis zu 15 Gew.-% schwerer siedende Komponenten.

## Patentansprüche

1. Destillationskolonne zur Auftrennung eines Mehrstoffgemisches in mindestens 3 Fraktionen, wobei die Destillationskolonne über mindestens eine Trennwand (2) verfügt und oberhalb der mindestens einen Trennwand (2) mindestens ein Stoffaustauschelement (1), in dem ein Stoffaustausch zwischen nach oben strömendem Dampf und nach unten strömender Flüssigkeit stattfindet, aufweist, **dadurch gekennzeichnet, dass** zwischen der mindestens einen Trennwand (2) und dem mindestens einen Stoffaustauschelement (1), im Falle des Vorhandenseins mehrerer Stoffaustauschelemente (1) zwischen der mindestens einen Trennwand (2) und dem untersten Stoffaustauschelement (1), eine Vorrichtung (10) zur Vermischung der links und rechts der mindestens einen Trennwand (2) nach oben strömenden Dampfströme angeordnet ist, wobei sich die Vorrichtung (10) in einem Bereich der Destillationskolonne, in dem die Dampfströme aus dem Bereich der mindestens einen Trennwand (2) nach oben strömen, ohne dabei auf herabströmende Flüssigkeit zu treffen, befindet, was dadurch realisiert wird, dass unterhalb des mindestens einen Stoffaustauschelements (1), im Falle des Vorhandenseins mehrerer Stoffaustauschelemente (1) unterhalb des untersten Stoffaustauschelements (1), ein Flüssigkeits-Sammler (7) zum Auffangen der aus dem mindestens einen Stoffaustauschelement (1) ablaufenden Flüssigkeit angeordnet ist, wobei der Flüssigkeits-Sammler (7) so ausgestaltet ist, dass die in diesem Flüssigkeits-Sammler (7) aufgefangene Flüssigkeit in einen Ringkanal (8) weitergeleitet und von dort
über einen Austrittsstutzen aus dem Inneren der Destillationskolonne heraus über außerhalb des Kolonnenkörpers befindliche Leitungen (11)
oder
über innerhalb des Kolonnenkörpers befindliche Leitungen
auf im Bereich der mindestens einen Trennwand (2) angeordnete Flüssigkeitsverteiler (5) und (6) gegeben wird.

2. Destillationskolonne gemäß Anspruch 1, bei der die Vorrichtung (10) zur Vermischung der links und rechts der mindestens einen Trennwand nach oben strömenden Dampfströme ausgewählt ist aus der Gruppe bestehend aus Mischerpackungen (10a), gewellten Blechlagen, bevorzugt ohne Löcher (10b), geraden Umlenkblechen (10c), schrägen Umlenkblechen (10d), Siebböden (10e), Kaminböden (10f), Glockenböden (10g), Ventilböden (10h), Statoren (10i), Rohre (10j), Kanälen (10k), Lochblechen (101) und Kombinationen der vorgenannten Vorrichtungen.

3. Destillationskolonne gemäß Anspruch 1 oder 2, bei der das oberhalb der Trennwand (2) angeordnete mindestens eine Stoffaustauschelement (1) ausgewählt ist aus der Gruppe bestehend aus Füllkörperschüttungen, strukturierten Stoffaustausch-Packungen, Glockenböden, Ventilböden, Siebböden und Kombinationen der vorgenannten Vorrichtungen.

4. Destillationskolonne gemäß einem der vorstehenden Ansprüche, bei der genau eine Trennwand (2) vorhanden ist.

5. Destillationskolonne gemäß einem der vorstehenden Ansprüche, bei der genau ein Stoffaustauschelement (1) vorhanden ist.

6. Destillationskolonne gemäß einem der vorstehenden Ansprüche, bei der das mindestens eine Stoffaustauschelement (1), im Falle des Vorhandenseins mehrerer Stoffaustauschelemente (1) mindestens das unterste Stoffaustauschelement (1), über den gesamten Querschnitt des Kolonnenkörpers reicht.

7. Verwendung einer Destillationskolonne gemäß einem der vorstehenden Ansprüche in der Reinigung von Isocyanaten.

8. Verwendung gemäß Anspruch 7, bei der das Isocyanat ausgewählt ist aus der Gruppe bestehend aus Toluylendiisocyanat, Methylendiphenyldiisocyanat, Hexamethylendiisocyanat, Pentamethylendiisocyanat, Isophorondiisocyanat, m-Xylylendiisocyanat und Dicyclohexylmethandiisocyanat.

9. Verwendung gemäß einem der Ansprüche 7 oder 8, bei der die Destillationskolonne gemäß einem der Ansprüche 1 bis 6 zur Feinreinigung des Isocyanats und Abtrennung polymerer Isocyanat-Fraktionen in einem Schritt eingesetzt wird.

10. Verwendung gemäß einem der Ansprüche 7 oder 8, bei der die Destillationskolonne gemäß einem der Ansprüche 1 bis 6 zur Auftrennung des Isocyanats in verschiedene Isomere eingesetzt wird.

## Claims

1. Distillation column for fractionating a multicomponent mixture into at least 3 fractions, wherein the distillation column comprises at least one dividing wall (2) and above the at least one dividing wall (2) comprises at least one mass transfer element (1) in which a mass transfer between ascending vapor and descending liquid takes place, **characterized in that** between the at least one dividing wall (2) and the at least one mass transfer element (1), in case of the presence of a plurality of mass transfer elements (1) between the at least one dividing wall (2) and the lowest mass transfer element (1), an apparatus (10) for mixing the vapor streams ascending left and right of the at least one dividing wall (2) is arranged, wherein the apparatus (10) is located in a region of the distillation column in which the vapor streams from the region of the at least one dividing wall (2) ascend without colliding with descending liquid, this being achieved by arranging below the at least one mass transfer element (1), in case of the presence of a plurality of mass transfer elements (1) below the lowest mass transfer element (1), a liquid collector (7) for collecting the liquid effluxing from the at least one mass transfer element (1), wherein the liquid collector (7) is configured such that the liquid collected in this liquid collector (7) is transferred into an annular channel (8) and from there is supplied
via an exit stub out from the inside of the distillation column via conduits (11) located outside the column body
or
via conduits located inside the column body t
o liquid distributors (5) and (6) arranged in the region of the at least one dividing wall (2).

2. Distillation column according to Claim 1, in which the apparatus (10) for mixing the vapor streams ascending left and right of the at least one dividing wall is selected from the group consisting of mixer packings (10a), corrugated sheet layers, preferably without holes (10b), straight baffle plates (10c), sloping baffle plates (10d), sieve trays (10e), chimney trays (10f), bubble-cap trays (10g), valve trays (10h), stators (10i), tubes (10j), channels (10k), perforated sheets (101) and combinations of the abovementioned apparatuses.

3. Distillation column according to Claim 1 or 2, in which the at least one mass transfer element (1) arranged above the dividing wall (2) is selected from the group consisting of dumped packing beds, structured mass transfer packings, bubble-cap trays, valve trays, sieve trays and combinations of the abovementioned apparatuses.

4. Distillation column according to any of the preceding claims, in which precisely one dividing wall (2) is present.

5. Distillation column according to any of the preceding claims, in which precisely one mass transfer element (1) is present.

6. Distillation column according to any of the preceding claims, in which the at least one mass transfer element (1), in case of the presence of a plurality of mass transfer elements (1) at least the lowest mass transfer element (1), extends over the entire cross section of the column body.

7. Use of a distillation column according to any of the preceding claims in the purification of isocyanates.

8. Use according to Claim 7, in which the isocyanate is selected from the group consisting of tolylene diisocyanate, methylenediphenyl diisocyanate, hexamethylene diisocyanate, pentamethylene diisocyanate, isophorone diisocyanate, m-xylylene diisocyanate and dicyclohexylmethane diisocyanate.

9. Use according to either of Claims 7 and 8, in which the distillation column according to any of Claims 1 to 6 is employed for fine purification of the isocyanate and removal of polymeric isocyanate fractions in one step.

10. Use according to either of Claims 7 and 8, in which the distillation column according to any of Claims 1 to 6 is employed for fractionation of the isocyanate into various isomers.

## Revendications

1. Colonne de distillation pour la séparation d'un mélange de plusieurs matières en au moins 3 fractions, la colonne de distillation étant équipée d'au moins une paroi de séparation (2) et comprenant au-dessus de ladite au moins une paroi de séparation (2) au moins un élément d'échange de matières (1), dans lequel un échange de matières a lieu entre la vapeur qui s'écoule vers le haut et le liquide qui s'écoule vers le bas, **caractérisée en ce qu'**entre ladite au moins une paroi de séparation (2) et ledit au moins un élément d'échange de matières (1), dans le cas de la présence de plusieurs éléments d'échange de matières (1) entre ladite au moins une paroi de séparation (2) et l'élément d'échange de matières (1) le plus inférieur, un dispositif (10) pour le mélange des courants de vapeur qui s'écoulent vers le haut à gauche et à droite de ladite au moins une paroi de séparation (2) est agencé, le dispositif (10) se trouvant dans une zone de la colonne de distillation dans laquelle les courants de vapeur s'écoulent vers le haut à partir de la zone de ladite au moins une paroi de séparation (2), sans rencontrer le liquide qui s'écoule vers le bas, ce qui est réalisé **en ce qu'**un collecteur de liquide (7) pour la réception du liquide qui s'écoule à partir dudit au moins un élément d'échange de matières (1) est agencé en dessous dudit au moins un élément d'échange de matières (1), dans le cas de la présence de plusieurs éléments d'échange de matières (1) en dessous de l'élément d'échange de matières (1) le plus inférieur, le collecteur de liquide (7) étant configuré de telle sorte que le liquide reçu dans ce collecteur de liquide (7) soit acheminé dans un canal circulaire (8) et à partir de celui-ci introduit
par le biais d'un raccord de sortie depuis l'intérieur de la colonne de distillation par des conduites (11) se trouvant à l'extérieur du corps de la colonne
ou
par des conduites se trouvant à l'intérieur du corps de la colonne,
dans des distributeurs de liquide (5) et (6) agencés dans la zone de ladite au moins une paroi de séparation (2).

2. Colonne de distillation selon la revendication 1, dans laquelle le dispositif (10) pour le mélange des courants de vapeur qui s'écoulent vers le haut à gauche et à droite de ladite au moins une paroi de séparation est choisi dans le groupe constitué par les garnissages de mélangeur (10a), les couches de tôles ondulées, de préférence sans trous (10b), les déflecteurs droits (10c), les déflecteurs inclinés (10d), les plateaux perforés (10e), les plateaux à cheminées (10f), les plateaux à calottes (10g), les plateaux à soupapes (10h), les stators (10i), les tubes (10j), les canaux (10k), les tôles perforées (10l) et les combinaisons des dispositifs susmentionnés.

3. Colonne de distillation selon la revendication 1 ou 2, dans laquelle ledit au moins un élément d'échange de matières (1) agencé au-dessus de la paroi de séparation (2) est choisi dans le groupe constitué par les chargements de corps de remplissage, les garnissages d'échange de matières structurés, les plateaux à calottes, les plateaux à soupapes, les plateaux perforés et les combinaisons des dispositifs susmentionnés.

4. Colonne de distillation selon l'une quelconque des revendications précédentes, dans laquelle exactement une paroi de séparation (2) est présente.

5. Colonne de distillation selon l'une quelconque des revendications précédentes, dans laquelle exactement un élément d'échange de matières (1) est présent.

6. Colonne de distillation selon l'une quelconque des revendications précédentes, dans laquelle ledit au moins un élément d'échange de matières (1), dans le cas de la présence de plusieurs éléments d'échange de matières (1) au moins l'élément d'échange de matières (1) le plus inférieur, s'étend sur la section transversale totale du corps de colonne.

7. Utilisation d'une colonne de distillation selon l'une quelconque des revendications précédentes dans la purification d'isocyanates.

8. Utilisation selon la revendication 7, dans laquelle l'isocyanate est choisi dans le groupe constitué par le diisocyanate de toluylène, le diisocyanate de diphénylméthylène, le diisocyanate d'hexaméthylène, le diisocyanate de pentaméthylène, le diisocyanate d'isophorone, le diisocyanate de m-xylylène et le diisocyanate de dicyclohexylméthane.

9. Utilisation selon l'une quelconque des revendications 7 ou 8, dans laquelle la colonne de distillation selon l'une quelconque des revendications 1 à 6 est utilisée pour la purification fine de l'isocyanate et la séparation de fractions isocyanate polymères en une étape.

10. Utilisation selon l'une quelconque des revendications 7 ou 8, dans laquelle la colonne de distillation selon l'une quelconque des revendications 1 à 6 est utilisée pour la séparation de l'isocyanate en différents isomères.
